# EUROPEAN PATENT SPECIFICATION

(11) **EP 0 608 425 B1**
(45) Date of publication and mention of the grant of the patent: **18.06.1997**
(21) Application number: 92921482.3
(22) Date of filing: 15.10.1992
(51) Int. Cl.: G01N 35/02

(54) **DEVICE FOR MEASURING VISCOSITY OF LIQUID**
VORRICHTUNG ZUM MESSEN DER VISKOSITÄT EINER FLÜSSIGKEIT
DISPOSITIF DE MESURE DE LA VISCOSITE D'UN LIQUIDE

(30) Priority: 18.10.1991 JP 271484/91
(43) Date of publication of application: 03.08.1994
(73) Proprietor: ALOKA CO., LTD., Mitaka-shi Tokyo 181 (JP); ABBOTT LABORATORIES, Abbott Park, Illinois 60064-3500 (US)
(72) Inventor: KAWANABE, Junichi, Mitaka-shi, Tokyo 181 (JP); TAKEDA, Masaaki, Mitaka-shi, Tokyo 181 (JP); KATAGI, Hitomi, Mitaka-shi, Tokyo 181 (JP); KATO, Yuko, Mitaka-shi, Tokyo 181 (JP); BIELARCZYK, Gregory, A., Lombard, IL 60148 (US); MAGEE, Rosie, L. c/o Ms. Annie Lewis, Oak Park, IL 60304 (US)
(74) Representative: Grünecker, Kinkeldey, Stockmair & Schwanhäusser Anwaltssozietät
(86) International application number: JP9201343
(87) International publication number: WO9308475

(56) References cited:
- FR-A- 2 604 271
- JP-A- 1 026 123
- JP-A- 2 222 826
- JP-A- 3 016 648
- JP-A- 3 099 245
- JP-A- 3 251 740
- JP-A-48 040 488
- JP-A-48 079 683
- JP-A-53 054 275
- JP-A-56 164 957
- JP-A-59 065 750
- JP-A-63 169 565
- US-A- 4 083 363
- PATENT ABSTRACTS OF JAPAN vol. 11, no. 219 (P-256) 16 July 1987 & JP-A-62 036 537 (KONISHIROKU) 17 February 1987
- PATENT ABSTRACTS OF JAPAN vol. 10, no. 9 (P-420) 14 January 1986 & JP-A-60 165 552 (TOSHIBA) 28 August 1985

## Description

### (Detailed Description of the Invention)

### (Industrial Field of Utilization)

The present invention relates to a liquid viscosity measuring device and more particularly to a liquid viscosity measuring device which enables a simultaneous measurement of a viscosity of a liquid sample with aspiration of the liquid sample in a pipetting apparatus or the like.

### [Prior Art]

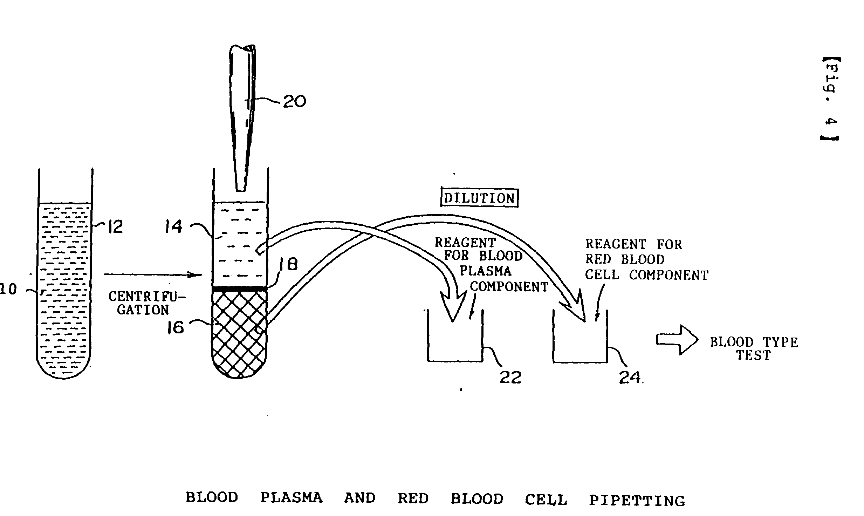

Various kinds of tests are conducted on a blood sample collected from a human body. For example, in a blood type test, as shown in Fig.4, a collected blood sample 10 is put into a test tube 12 and then separated into a blood plasma component 14 and a red blood cell component 16 by centrifugation. Practically, a small quantity of white blood cell component 18 appears between the blood plasma component 14 and the red blood cell component 16.

A blood sample pipetting method which is carried out in a conventional pipetting apparatus generally comprises two processes, including a process of pipetting blood plasma and a process of pipetting red blood cells. In the blood plasma pipetting process, the blood plasma component 14 is aspirated through a nozzle tip 20, and then dispensed into a plurality of other recipient containers 22 in a predetermined volume, respectively. In the red blood cell pipetting process, the red blood cell component 16 is aspirated through the nozzle tip 20, and then transferred to a diluting container (not shown) to be mixed with a diluent. Thereafter, the diluted red blood cell component 16 is aspirated again through the nozzle tip 20 and then dispensed into a plurality of other recipient containers 24, respectively in a predetermined volume.

Blood type testing reagents (i.e., a reagent for the blood plasma component and a reagent for the red blood cell component) are then introduced into the recipient containers 22, 24, respectively.

Subsequently, these recipient containers 22, 24 are conveyed to an agglutination testing apparatus, where agglutination of the samples in the containers 22, 24 are measured optically. On the basis of the results of the measurements, A type, B type, 0 type or AB type, or Rh type, or the like is determined.

The viscosity of the red blood cell component is useful data for diagnosing diseases, and it is also a useful factor for determining an aspiration pressure to be set when pipetting the blood sample.

An apparatus for determining the blood viscosity in vivo is disclosed in US-A-4 083 363. However, in conventional pipetting apparatuses, there has been equipped with no viscosity measuring device, and the viscosity of the blood sample or the like has been measured by another measuring apparatus.

### (Problem to be solved by the Invention)

As aforementioned, in the conventional pipetting apparatuses it has been impossible to measure the viscosity of the blood sample simultaneously with the pipetting operation of the blood sample. Therefore, means for enabling a simultaneous measurement of the viscosity of the blood sample during the pipetting operation has been so far desired.

As an apparatus for measuring the viscosity of the liquid sample, a capillary viscometer, a rotational viscometer, a falling body viscometer, an oscillational viscometer, and the like are conventionally well known. However, it takes a long time to measure the viscosity with these conventional viscometers, and their operations are troublesome. Further, these viscometers require to prepare a predetermined volume of the blood sample to be used for the viscosity measurement, in addition to the blood sample to be used for the pipetting. Therefore, when measuring the viscosity of the blood sample by the conventional viscometers, there is a disadvantage in that recycling of the blood sample used for the viscosity measurement is difficult.

This invention has been made in view of above problems. It is therefore an object of the present invention to provide a liquid viscosity measuring device capable of measuring a viscosity of a liquid sample simultaneously with the aspiration of the liquid sample.

Another object of the present invention is to provide a pipetting apparatus which enables simultaneous viscosity measurement during the pipetting operation, without preparing another blood sample to be used only for the viscosity measurement.

### (Means for Solving the Problems)

According to the present invention, the aforementioned object can be achieved by a liquid viscosity measuring device comprising: a viscosity table storing data corresponding to relationship between a viscosity of a liquid sample and a time required from a point of time when the liquid sample is aspirated under a predetermined initial aspiration pressure to a point of time when the aspiration pressure of an aspiration system reaches a predetermined value; and a viscosity measuring unit for measuring the required time for the liquid sample, and comparing the required time with the data in said table, thereby obtaining the viscosity of the liquid sample.

According to the present invention, the aforementioned object can be also achieved by a pipetting apparatus having a liquid sample aspirating device including a nozzle tip for aspirating a liquid sample, an aspirating pump connected to the nozzle tip, and a pressure sensor for measuring a pressure in an aspiration system, wherein the pipetting apparatus further comprises a liquid viscosity measuring device as defined in Claim 1 which measures the viscosity of the liquid sample simultaneously with the aspiration of the liquid sample.

### (Operation of the Invention)

According to the aforementioned construction, a viscosity table representing the relationship between the required time and the viscosity of the liquid sample is previously prepared. The viscosity measuring unit measures the required time for the liquid sample to obtain the viscosity of the liquid sample with reference to the data stored in the viscosity table.

Therefore, it is possible to measure the viscosity of the liquid sample simultaneously with the aspiration of the liquid sample.

If the apparatus according to the present invention is applied to a pipetting apparatus, there are advantages as follows. The viscosity of the blood sample can be measured simultaneously with the pipetting operation of the blood sample. Further, it is possible to measure the viscosity, without preparing a predetermined volume of a blood sample to be used only for the viscosity measurement.

The principles of the present invention will now be described with reference to the accompanying drawings.

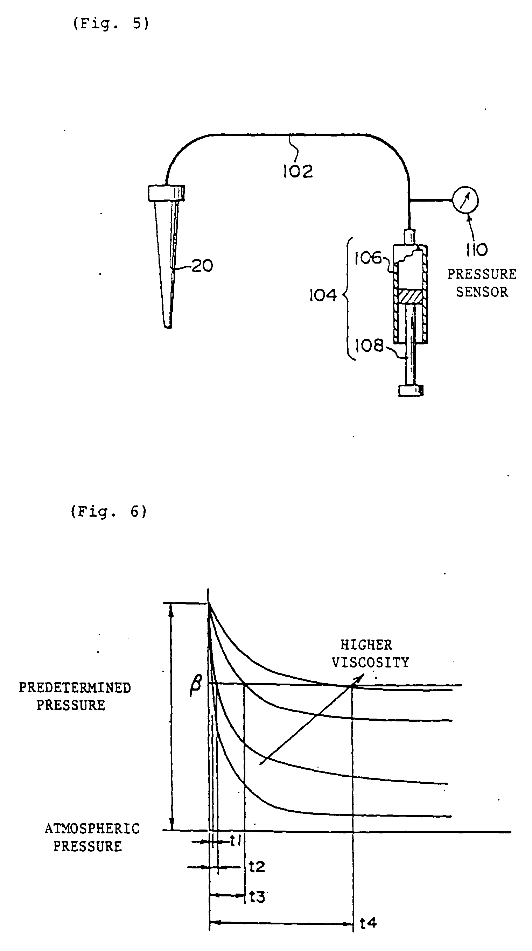

Fig. 5 shows schematically a construction of an ordinary aspirating device used in a pipetting apparatus or the like. A nozzle chip 20 is connected to a pump 104 via an air hose 102. The pump 104 is composed of a cylinder 106 and a piston 108. The air hose 102 is equipped with a pressure sensor 110 for detecting an internal pressure of the air hose 102.

Fig. 6 shows changes of the pressure in the aspiration system which are detected by the pressure sensor 110, after the initial aspiration pressure has been produced by pulling downwardly the piston 108 as shown in Fig. 5. As shown in Fig.6, as the viscosity becomes higher, the time required until the pressure returns from a predetermined pressure to an atmospheric pressure becomes longer. This means that if a predetermined pressure value is determined as β, the time required until a produced pressure reaches the predetermined value β differs depending on the viscosity of a liquid sample to be aspirated.

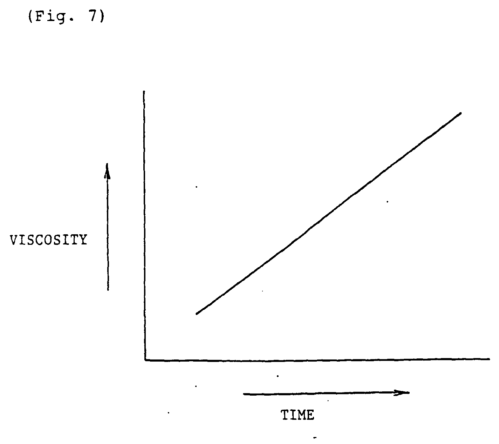

Fig. 7 shows the relationship between the required time and the viscosity. The relationship therebetween is approximately proportional. This relationship can be also proved by calculation.

If the predetermined pressure value β is set, the table representing the relationship shown in Fig. 7 is prepared, and the required time is measured, then it is easy to obtain the viscosity. Namely, according to the present invention, the required time is measured and the viscosity is obtained on the basis of the measured result.

### [Embodiment]

Embodiments of the present invention will now be described with reference to the accompanying drawings.

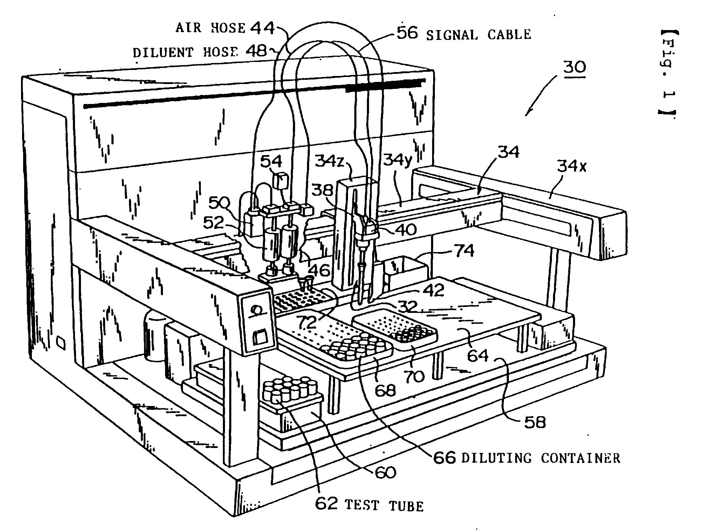

Fig. 1 is a perspective view showing schematically a pipetting apparatus 30 according to the present invention.

In this embodiment, the pipetting apparatus 30 is used to pipette the blood plasma component and the red blood cell component which have been obtained by centrifugation to perform a preprocess for blood type test.

As shown substantially in the roughly central portion of Fig. 1, a nozzle 32 for aspirating a blood sample is held by an XYZ robot 34 so as to be movable three-dimensionally.

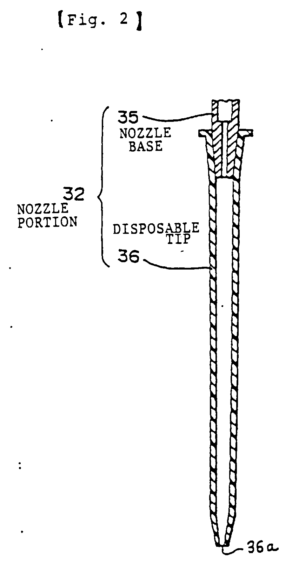

Fig. 2 shows a cross sectional view of a main part of the nozzle 32. The nozzle 32 is composed of a nozzle base 35, and a disposable tip serving as a nozzle tip 36. Thus, the pipetting apparatus in the embodiment of the present invention uses a disposable type nozzle tip. A distal end of the nozzle base 35 is forced into an upper opening of the nozzle tip 36 and is fitted therein. Thus, the nozzle tip 36 is fixed firmly to the nozzle base 35. The nozzle tip 36 has at its lower end a smaller orifice 36a from which the blood sample is aspirated and dispensed. The nozzle tip 36 may be made of a hard plastic material or the like, and the nozzle base 35 may be made of a metal.

In Fig. 1, the XYZ robot 34 is composed of an X drive portion 34x, an Y drive portion 34y and a Z drive portion 34z. On the Z drive portion 34z, an elevator 38 equipped with the nozzle 32 is mounted so as to be vertically movable. The elevator 38 has a limit switch 40 serving as a jamming sensor or the like.

The limit switch 40 detects an external force imparted upwardly to the nozzle 32 and having a value greater than a predetermined force.

Onto the Z drive portion 34z, a diluent pipette 42 for dispensing a diluent is fixedly mounted. An air hose 44 is connected at one end thereof to the nozzle 32 and at the other end thereof to a syringe 46 serving as a pump for causing aspirating and dispensing actions. A diluent hose 48 is connected at one end thereof to the diluent pipette 42 and at the other end thereof to a syringe 52 via an electromagnetic valve 50.

Between the syringe 46 and the nozzle 32, a pressure sensor 54 for measuring an internal pressure of the air hose 44 is connected. A signal from the limit switch 40 is fed to the apparatus via a cable 56.

On a test tube rack 60 placed on a pipetting table 58, a plurality of test tubes 62 each containing a blood sample which has been already subjected to centrifugation are held uprightly. Each test tube 62, as shown in Fig.4, contains the blood sample in which the blood plasma component and the red blood cell component are separated into an upper portion and a lower portion in the test tube 62, respectively. On a horizontal table 64 mounted on the pipetting table 58, there are provided a dilution tray 68 equipped with a plurality of diluting containers 66, and a microplate 70. On the microplate 70, there are provided a plurality of wells each serving as a recipient container for containing the blood plasma component or the diluted the red blood cell component. After all of the blood samples have been pipetted, the microplate 70 is conveyed to an apparatus for blood type test, by which an agglutination test, for example, is made optically. Further, the agglutination test may be made visually.

In the device according to the present invention, the nozzle tip is a disposable type. Therefore, a plurality of new nozzle tips are prepared on a nozzle tip stand 72, and the nozzle tip already used is exchanged with a new one. There is also provided a nozzle scrap tray 74.

Therefore, in the device according to the present invention, it is possible to aspirate the blood plasma component or the red blood cell component through the nozzle tip 36 of the nozzle 32 and then transfer it into other recipient container. This device may also be applied to purposes other than pipetting of the blood sample. Various kinds of applications are possible.

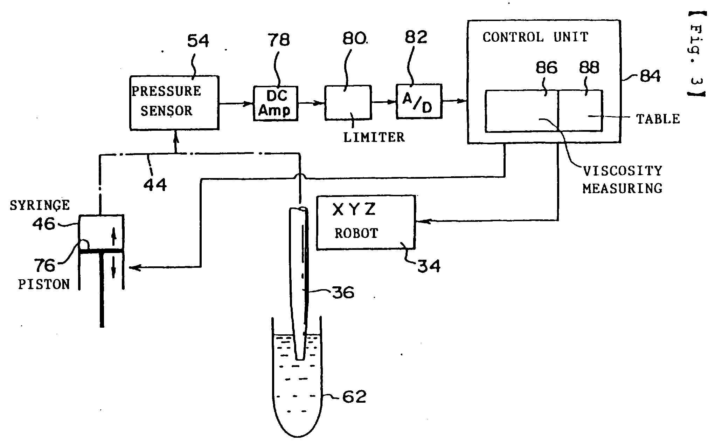

Fig. 3 is a block diagram which shows diagrammatically the structure of the device of the embodiment according to the present invention. By moving a piston 76 up and down, inside volume of the syringe 46 varies, so that an aspirating pressure or a dispensing pressure is transmitted to the nozzle tip 36 of the nozzle 32 via the air hose 44 to perform the aspiration or dispensation of the blood sample. The internal pressure of the air hose 44 is detected by the pressure sensor 54, a sensor signal outputted from the pressure sensor 54 is amplified by a DC amplifier 78 and is then fed to an analog-digital converter 82 via a limiter circuit 80. The limiter circuit 80 functions as a protection circuit for suppressing any excessive input. The analog-digital converter 82 converts the sensor signal into a digital signal and feeds the digital signal to a control unit 84.

The control unit 84 includes a computer, for example, for controlling the inside volume of the syringe 46, and the XYZ robot 34, etc. In this embodiment, the control unit 84 also includes a viscosity measuring unit 86 and a table 88. The detailed of the viscosity measuring unit 86 and the table 88 will be described below.

As aforementioned, when a liquid is aspirated, a predetermined pressure used as an initial aspiration pressure is produced. In this case, there is a close relationship between the liquid viscosity and a time (required time) required from a point of time when the predetermined pressure is produced, to a point of time when the aspiration pressure reaches a certain pressure value (β).

In view of the above, in the embodiment according to the present invention, the data corresponding to the relationship between the viscosity and the required time is stored in the table 88.

Specifically, when the blood sample is aspirated by the tip 36 in Fig. 3, a predetermined initial aspiration pressure is produced by the pump under the monitoring of the control unit 84, and subsequent pressure changes are detected by the pressure sensor 54. The viscosity measuring unit 86 measures a time required from a point of time when the initial aspiration pressure is produced, to a point of time when the pressure reaches a predetermined pressure value (β). Then, the viscosity measuring unit 86 compare the measured time with the data storing in the table 88 to obtain the viscosity of the blood sample. The obtained viscosity is displayed in a display unit (not-shown), and the data representing the obtained viscosity is supplied to the control unit 84 and used for controlling the pump.

The viscosity measuring unit 86 as described above has advantages as follows. Since the viscosity can be measured simultaneously with the aspiration of the blood sample, the unit 86 requires no additional time for measuring the viscosity, and realizes a very simple viscosity measurement. Further, it is unnecessary to prepare an additional blood sample specially for viscosity measurement.

### (Advantageous Effects of the Invention)

According to the present invention, since it is possible to easily measure the viscosity of the liquid sample by measuring the required time, there is an advantage of enabling a simultaneous viscosity measurement during the aspiration of the liquid sample. Therefore, the viscosity measuring apparatus according to the present invention is particularly useful when employed in the pipetting apparatus for pipetting the blood sample or the like.

### (Brief Explanation of the Drawings)

Fig. 1 is a perspective view showing schematically an embodiment of a pipetting apparatus according to the present invention;
Fig. 2 is a cross-sectional view showing a main part of a nozzle;
Fig. 3 is a block diagram showing schematically the pipetting apparatus of Fig. 1
Fig. 4 is an explanatory view showing a pipetting operation of blood plasma and red blood cells for a preprocess of a blood type test;
Fig. 5 is a schematic view showing an ordinary aspirating device;
Fig. 6 is a characteristic graph showing the relationship between the elapsed time after an initial aspiration pressure is produced, and the pressure changes in an aspirating system, for respective liquid samples having different viscosities; and
Fig. 7 is an explanatory view showing the relationship between the required time and the viscosity.

### [Explanation of References Numerals]

- 30: pipetting apparatus
- 32: nozzle
- 34: XYZ robot
- 35: nozzle base
- 36: disposable tip
- 54: pressure sensor
- 84: control unit
- 86: viscosity measuring unit
- 88: table

## Claims

1. A liquid viscosity measuring device (84) comprising:
(a) a viscosity table (88), storing data corresponding to the relationship between a viscosity of a liquid sample and a time required for an aspiration system to reach, from a point of time when the liquid sample is aspirated under a predetermined initial aspiration pressure, a predetermined aspiration pressure (β); and
(b) a viscosity measuring unit (86) for measuring said required time for the liquid sample, and comparing said time with the data in said table (88) to thereby obtain the viscosity of the liquid sample.

2. A pipetting apparatus (30) having a liquid sample aspirating device including a nozzle tip (36) for aspirating a liquid sample, an aspirating pump (46, 76) connected to the nozzle tip (36), and a pressure sensor (54) for measuring a pressure in an aspiration system, wherein the pipetting apparatus (30) further comprises a liquid viscosity measuring device (84) as defined in Claim 1 to measure the viscosity of a liquid sample simultaneously with the aspiration of the liquid sample.

## Patentansprüche

1. Vorrichtung (84) zum Messen der Viskosität einer Flüssigkeit mit
(a) einer Viskositätstabelle (88), die Daten speichert, die dem Verhältnis zwischen einer Viskosität einer Flüssigkeitsprobe und einer Zeitdauer entsprechen, die von einem Ansaugsystem von einem Zeitpunkt, zu dem die Flüssigkeitsprobe mit einem vorbestimmten Ausgangsansaugdruck angesogen wird, bis zur Erreichung eines vorbestimmten Ansaugdruckes (β) benötigt wird, und
(b) einer Viskositätsmeßeinheit (86), die die benötigte Zeit für die Flüssigkeitsprobe mißt und diese Zeit mit den Daten in der Tabelle (88) vergleicht, wodurch die Viskosität der Flüssigkeitsprobe ermittelt wird.

2. Pipettiervorrichtung (30) mit einer Ansaugvorrichtung für eine Flüssigkeitsprobe mit einer Düsenspitze (36) zum Ansaugen einer Flüssigkeitsprobe, einer Ansaugpumpe (46,76), die mit der Düsenspitze (36) verbunden ist, und einem Druckmeßfühler (54) zum Messen des Druckes in einem Ansaugsystem, wobei die Pipettiervorrichtung (30) außerdem eine Vorrichtung (84) zum Messen der Viskosität einer Flüssigkeit gemäß Anspruch 1 aufweist, um die Viskosität einer Flüssigkeitsprobe gleichzeitig mit dem Ansaugen der Probe zu messen.

## Revendications

1. Dispositif de mesure (84) de la viscosité d'un liquide comprenant :
(a) une table de viscosité (88) qui stocke des données correspondant à la relation entre une viscosité d'un échantillon de liquide et une durée nécessaire pour qu'un système d'aspiration atteigne, à partir d'un instant auquel l'échantillon de liquide est aspiré sous une pression d'aspiration initiale prédéterminée, une pression d'aspiration prédéterminée (β) ; et
(b) une unité de mesure de la viscosité (86) pour mesurer ladite durée nécessaire pour l'échantillon de liquide, et comparer ladite durée avec les données contenues dans ladite table (88) pour ainsi obtenir la viscosité de l'échantillon de liquide.

2. Appareil à pipetter (30) comportant un dispositif d'aspiration d'échantillon de liquide comprenant une pointe de pipette (36) pour aspirer un échantillon de liquide, une pompe d'aspiration (46, 76) reliée à la pointe de pipette (36), et un capteur de pression (54) pour mesurer une pression dans un système d'aspiration, l'appareil à pipetter (30) comprenant en outre un dispositif de mesure (84) de la viscosité tel que défini dans la revendication 1, pour mesurer la viscosité d'un échantillon de liquide simultanément à l'aspiration de l'échantillon de liquide.
